# FASCICULE DE BREVET EUROPEEN

(11) **EP 2 591 085 B1**
(45) Date de publication et mention de la délivrance du brevet: **12.04.2017**
(21) Numéro de dépôt: 11743841.6
(22) Date de dépôt: 08.07.2011
(51) Int. Cl.: C12Q 1/24, C12M 1/26

(54) **PROCEDE DE PRELEVEMENT ET/OU DEPÔT D'UN ECHANTILLON DE MATIERE BIOLOGIQUE ET DISPOSITIF METTANT EN OEUVRE UN TEL PROCEDE**
VERFAHREN ZUR ENTNAHME UND UND/ODER ABSCHEIDUNG EINER BIOLOGISCHEN PROBE UND VORRICHTUNG ZUR UMSETZUNG DES VERFAHRENS
METHOD FOR COLLECTING AND/OR DEPOSITING A SAMPLE OF BIOLOGICAL MATERIAL, AND DEVICE IMPLEMENTING SAID METHOD

(30) Priorité: 08.07.2010 FR 1055565
(43) Date de publication de la demande: 15.05.2013
(73) Titulaire: Biomérieux, 69280 Marcy L'etoile (FR)
(72) Inventeur: CHARRIER, Jean-Philippe, F-69160 Tassin la Demi-Lune (FR); COLIN, Bruno, F-69280 Marcy l'Etoile (FR); DRAZEK, Laurent, F-38100 Grenoble (FR); PARIS, Cécile, F-69690 Bessenay (FR); RAYMOND, Jean-Claude, F-69690 Bessenay (FR); DECAUX, Dominique, F-69630 Chaponost (FR)
(86) Numéro de dépôt international: PCT/FR2011/051631
(87) Numéro de publication internationale: WO 2012/004545

(56) Documents cités:
- EP-A1- 0 152 368
- EP-A1- 1 291 413
- WO-A1-92/12233
- WO-A1-2005/018461
- FR-A1- 2 527 221
- US-A1- 2004 267 181

## Description

La présente invention a pour objet un procédé de prélèvement et un procédé de dépôt d'un échantillon de matière biologique brut ou enrichi cultivé au contact d'un milieu de culture, ainsi qu'un dispositif mettant en oeuvre ces procédés.

Actuellement, le prélèvement d'un échantillon de microorganismes (bactéries, moisissures, levures ou similaires) cultivés sur milieu de culture gélosé en boîte de Pétri, ou sur tout autre support, est réalisé grâce à des automates tel que décrits dans les demandes de brevet FR 2 517 221 A1 et WO 92/122333 A1 et/ou à l'aide d'outils à usage unique tels qu'öses, bâtonnets, tubes ou encore cônes

Cependant, ces automates et consommables ne permettent pas de prélever de façon sure et efficace tous les types de microorganismes car ces derniers peuvent revêtir des formes, tailles, consistances, structures ou aspects très divers.

D'autre part, ces consommables ne permettent ni un dépôt optimal de la matière biologique sur des supports d'analyse tels que des plaques, ni une remise en suspension facile de ladite matière biologique.

De plus, il est important de pouvoir permettre le prélèvement d'une colonie bactérienne ou d'une fraction de cette colonie sans prélever le milieu de culture, situé sous la colonie : ce qui pourrait fausser les résultats d'analyse.

La qualité des résultats d'analyse dépend également de la concentration du dépôt en matière biologique formé à partir de l'échantillon prélevé, ainsi que de son homogénéité sur le support sur lequel il est déposé.

La présente invention est définie par l'objet des revendications. La présente invention a pour but de résoudre tout ou partie des inconvénients mentionnés ci-dessus.

A cet effet, la présente invention a pour objet un procédé de prélèvement de tout ou partie d'un échantillon de matière biologique, brut, enrichi ou cultivé au contact d'un milieu de culture, éventuellement gélosé, utilisant une sonde pourvue d'une extrémité de terminaison, ledit procédé de prélèvement comprenant les étapes de refroidissement de l'extrémité de terminaison de la sonde, collage de tout ou partie de l'échantillon de matière biologique à prélever par contact de l'extrémité de terminaison sur l'échantillon, de matière biologique ou par l'application d'une pression exercée par l'extrémité de terminaison sur l'échantillon de matière biologique, et prélèvement de tout ou partie de l'échantillon de matière biologique de façon à séparer l'échantillon de matière biologique de son support, tel qu'un milieu de culture.

Ce procédé permet de prélever tout type d'échantillon de matière biologique cultivée in vitro quelles que soient leurs formes et leurs consistances. De plus, par ce procédé de prélèvement, il n'y a pas de contamination possible de la sonde car son extrémité, en contact avec l'échantillon, peut être stérilisée après chaque utilisation ou alors n'est jamais en contact direct avec l'échantillon.

De plus, par ce procédé de prélèvement, il n'y a pas nécessairement besoin d'utiliser une extrémité de terminaison à usage unique, ce qui réduit le coût d'utilisation tout en permettant des cycles d'utilisation plus rapides (meilleure productivité du dispositif).

Selon un moyen de mise en oeuvre, la sonde est une sonde cryogénique comprenant des moyens pour être refroidie.

Selon une mise en oeuvre du procédé de prélèvement, préalablement à l'étape de collage, il est réalisé une étape de mouillage de l'extrémité de terminaison de la sonde dans une solution liquide, telle de l'eau distillée afin de former une couche de glace sur l'extrémité de terminaison.

Cette disposition permet de créer un consommable en extemporané permettant de coller l'échantillon afin de le prélever. De plus, par cette mise en oeuvre du procédé de prélèvement, l'échantillon n'est jamais directement en contact avec l'extrémité de terminaison de la sonde.

Selon une mise en oeuvre du procédé, préalablement à l'étape de collage, l'échantillon de matière biologique à prélever est recouvert d'une solution liquide, afin de former une couche de glace autour de l'échantillon lors de l'étape de collage de tout ou partie dudit échantillon avec l'extrémité de terminaison de la sonde.

Selon une mise en oeuvre du procédé de prélèvement, la solution liquide est prise dans le groupe comprenant l'eau, une solution saline, un tampon, un milieu de culture liquide ou une matrice habituellement utilisée pour ioniser l'échantillon de matière biologique en vue de son analyse par un dispositif de mesure tel un spectromètre de masse. Un tampon peut par exemple un tampon carbonate (10 à 100 mmol/L, idéalement 25 mmol/L).

Cette disposition permet de réduire d'une étape le procédé d'analyse de l'échantillon par un spectromètre de masse, la matrice ayant la double fonction d'être utilisée pour l'étape de collage lors du prélèvement et pour une étape de préparation de l'échantillon en vue de son analyse par un spectromètre de masse.

Alternativement, la solution liquide peut être un milieu de culture de microorganismes.

Selon une mise en oeuvre du procédé de prélèvement, l'étape de mouillage de l'extrémité de terminaison de la sonde ou de l'échantillon de matière biologique à prélever dans une solution liquide est répétée successivement au moins deux fois, afin de former une superposition de couches de glace, telle une stalactite.

Cette disposition permet d'agrandir la surface de collage de la stalactite ainsi créée et ainsi de pouvoir prélever des échantillons de plus grande taille.

Selon une mise en oeuvre du procédé de prélèvement, l'extrémité de terminaison est amovible.

Cette disposition permet d'augmenter considérablement la surface utile de collage de l'extrémité de terminaison.

Selon une mise en oeuvre du procédé de prélèvement, l'extrémité de terminaison possède des propriétés ferromagnétiques.

Selon une mise en oeuvre du procédé de prélèvement, l'étape de prélèvement de tout ou partie de l'échantillon de matière biologique consiste à appliquer un champ magnétique, par exemple à l'aide d'un électro-aimant, au voisinage de l'extrémité de terminaison ferromagnétique de façon à attirer l'extrémité de terminaison et ainsi la récupérer.

Cette disposition permet d'automatiser le prélèvement sans risquer de contaminer l'échantillon prélevé.

La présente invention a également pour objet un procédé de dépôt dans un contenant ou sur une plaque d'analyses de tout ou partie d'un échantillon de matière biologique collé sur une extrémité de terminaison givrée d'une sonde, ledit procédé de dépôt comportant une étape de séparation de l'extrémité de terminaison de la sonde et de tout ou partie de l'échantillon de matière biologique, collé sur ladite extrémité de terminaison.

Cette disposition permet d'exploiter l'ensemble ou partie de l'échantillon.

Selon une mise en oeuvre du procédé de dépôt, l'étape de séparation est réalisée sans contact de l'échantillon de matière biologique collé sur l'extrémité de terminaison givrée avec le contenant ou la plaque d'analyses.

Dans ce cas, l'étape de séparation de l'extrémité de terminaison et de tout ou partie de l'échantillon de matière biologique collé sur ladite extrémité de terminaison est réalisée par l'application d'un choc mécanique sur l'extrémité de terminaison.

Cette disposition permet de recueillir immédiatement dans un état gelé l'ensemble de l'échantillon prélevé.

Selon une mise en oeuvre du procédé de dépôt, l'étape de séparation de l'extrémité de terminaison et de l'ensemble ou partie de l'échantillon de matière biologique collé sur l'extrémité de terminaison givrée est réalisée par un moyen de chauffage ou à l'air ambiant.

Cette disposition permet de recueillir l'échantillon dans un état liquide et permet de distribuer plusieurs parties d'échantillon sur plusieurs supports différents.

Selon une mise en oeuvre du procédé de dépôt, l'étape de séparation est réalisée par le contact séquentiel de l'échantillon de matière biologique avec le contenant ou la plaque d'analyses, ce contact provoquant la fonte d'une couche superficielle de glace et le dépôt de matière biologique.

Cette disposition permet d'obtenir un contrôle sur la quantité d'échantillon déposé et sur le choix du support de dépôt, de plus la fonte de la couche superficielle permet d'obtenir un dépôt homogène, tant en termes de répartition de matière biologique, que d'épaisseur de la couche déposée.

Selon une mise en oeuvre du procédé de dépôt, tout ou partie de l'échantillon de matière biologique est distribué sur une plaque d'analyses de manière à réaliser plusieurs dépôts distincts de parties d'un même échantillon de matière biologique, collé sur l'extrémité de terminaison.

Cette disposition permet de faire plusieurs mesures sur des dépôts provenant d'un même échantillon, chacun de ces dépôts étant homogènes, ce qui permet d'améliorer la qualité de certains spectres de mesure, en particulier pour des mesures de spectrométrie de masse pour laquelle une faible homogénéité du dépôt conduit à des spectres comprenant des pics peu intenses avec un signal comprenant beaucoup de bruit.

Selon une mise en oeuvre du procédé de dépôt, le dépôt est effectué par le contact continu par exemple sous forme de traits de tout ou partie de l'échantillon de matière biologique, collé sur l'extrémité de terminaison, avec le contenant ou la plaque d'analyses.

Cette disposition permet de réaliser un gradient de concentration de matière biologique sur le support de dépôt puis de focaliser les mesures sur la zone du gradient qui permet d'obtenir les meilleurs résultats, par exemple pour des mesures de spectrométrie de masse.

La présente invention a également pour objet un dispositif de prélèvement et de dépôt de tout ou partie d'un échantillon de matière biologique, brut, enrichi ou cultivé au contact d'un milieu de culture et destiné à être déposé dans un contenant ou une plaque d'analyses, caractérisé en ce qu'il comprend une sonde pourvue d'une extrémité de terminaison, un moyen de refroidissement destiné à givrer l'extrémité de terminaison, des moyens d'entraînement destinés à exercer une pression de la sonde sur l'échantillon de manière à congeler tout ou partie de l'eau contenue dans l'échantillon afin de le coller à l'extrémité de terminaison, à séparer tout ou partie de l'échantillon de son support, tel qu'un milieu de culture, à rapprocher tout ou partie de l'échantillon du contenant ou de la plaque d'analyses.

Cette disposition permet de disposer d'un dispositif automatisé et réutilisable pouvant effectuer plusieurs prélèvement et dépôt en un minimum de temps.

Selon un mode de réalisation, le dispositif comprend un moyen de chauffage destiné à décoller l'échantillon de l'extrémité de terminaison.

Selon un mode particulier de réalisation, le moyen de chauffage est destiné à stériliser l'extrémité de terminaison. Une telle stérilisation est réalisation avant tout de prélèvement.

Avantageusement, le moyen de refroidissement et le moyen de chauffage sont constitués par au moins un élément Peltier. Avantageusement, plusieurs éléments Peltier superposés sont utilisés.

Cette disposition permet de faire passer plus rapidement l'échantillon à l'état liquide et facilite sa remise en suspension et son homogénéisation.

Selon un mode de réalisation, l'extrémité de terminaison est métallique ou minérale.

Cette disposition permet d'absorber plus vite la chaleur contenue dans l'eau de l'échantillon et donc de le congeler plus rapidement.

Avantageusement, l'extrémité de terminaison est au moins partiellement recouverte d'une revêtement ou traitement hydrophobe. Un tel revêtement permet une évacuation facilitée et optimisée du liquide et/ou de l'échantillon présent sur l'extrémité de terminaison, un fois celui-ci dégelé.

Selon un mode de réalisation, l'extrémité de terminaison est amovible.

Cette disposition permet de désolidariser l'extrémité de terminaison givrée afin de la coller directement à l'échantillon de matière biologique à prélever.

Elle permet par ailleurs, à l'extrémité de terminaison d'être stockée dans un environnement réfrigéré, ce qui peut être particulièrement avantageux, si l'on souhaite réalisée une analyse extemporanément.

Selon, un mode de réalisation, l'extrémité de terminaison présente une forme optimisant le collage de tout ou partie de l'échantillon de matière biologique à prélever ou de solution liquide. Selon un mode particulier de réalisation, l'extrémité de terminaison comprend une extrémité en pointe. Avantageusement, la forme de l'extrémité de terminaison peut être de forme tronconique. Une telle forme facilite grandement le collage de l'échantillon ou de la solution liquide lors du mouillage.

Cette disposition permet un prélèvement précis de l'échantillon mais également le prélèvement de très petits échantillons tels que des microcolonies bactériennes.

Selon un mode de réalisation, le dispositif comprend au moins un capteur contrôlant la pression exercée par les moyens d'entraînement par l'intermédiaire de la sonde en contact avec l'échantillon de matière biologique à prélever cultivé sur le milieu de culture, et ce afin de stopper cette pression.

Cette disposition permet de se prémunir de tout collage du support de l'échantillon, tel qu'un milieu de culture gélosé.

Selon un mode de réalisation, le capteur est un capteur de pression ou de force, ce capteur pouvant par exemple être placé sous un support de boîte de Pétri.

Cette disposition permet de contrôler la quantité de matière biologique prélevée, à la manière d'une balance électronique.

Selon un mode de réalisation, le dispositif comprend un capteur détectant le contact entre l'extrémité de terminaison et la matière biologique afin d'éviter toute pression.

Selon un mode de réalisation, le capteur est un capteur électrique binaire.

Cette disposition permet à moindre coûts de se prémunir de tout collage du support de l'échantillon, tel qu'un milieu de culture en gélose.

La présente invention a également pour objet un kit comprenant un dispositif tel que décrit précédemment comportant une pluralité d'extrémités de terminaison interchangeables.

Ces extrémités de terminaison peuvent être à usage unique ou pas. Elles peuvent être en forme de pointe, de boucle ou encore de cylindre et de dimensions différentes.Cette disposition permet de disposer d'extrémités de terminaison de formes et de tailles différentes pour un même dispositif, afin de s'adapter à la taille de l'échantillon à prélever.

La présente invention a également pour objet un appareil d'analyse biologiques comprenant un dispositif ou un kit tels que décrits précédemment.

De toute façon, l'invention sera bien comprise à l'aide de la description qui suit, en référence aux dessins schématiques annexés représentant, à titre d'exemple non limitatif, un dispositif mettant en oeuvre les étapes d'un procédé selon l'invention.
La figure 1 montre le schéma synoptique d'un dispositif selon l'invention.
La figure 2 montre quelques étapes d'un procédé de prélèvement selon un premier moyen de mise en oeuvre selon l'invention.
La figure 3 montre quelques étapes d'un procédé de prélèvement selon un deuxième moyen de mise en oeuvre selon l'invention.
La figure 4 illustre plusieurs exemples de prélèvement et de dépôt de l'ensemble ou partie d'un échantillon.
La figure 5 illustre un mode de réalisation des moyens de refroidissement de l'extrémité de terminaison par des éléments Peltier.
La figure 6 montre un spectre de masse obtenu après prélèvement et dépôt d'une colonie de *Staphylococcus aureus* utilisant les procédés selon un premier mode de réalisation de l'invention.
La figure 7 montre un spectre de masse obtenu après prélèvement et dépôt d'une colonie d'*Escherichia coli* utilisant les procédés selon le premier mode de réalisation de l'invention.
La figure 8 montre un spectre de masse obtenu après prélèvement et dépôt d'une colonie d'*Escherichia coli* utilisant les procédés selon un deuxième mode de réalisation de l'invention.

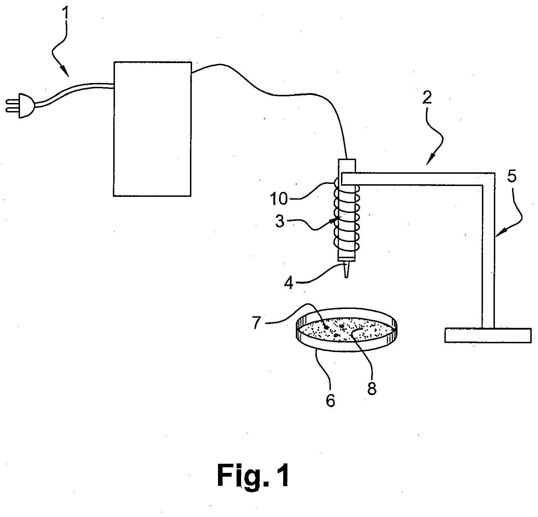

Comme illustré à la figure 1, un appareil de prélèvement et de dépôt 1 selon l'invention comprend un dispositif de prélèvement et de dépôt 2.

Ce dispositif de prélèvement et de dépôt 2 comporte une sonde 3 ainsi que des moyens d'entraînement 5 destinés à déplacer la sonde 3 dans l'espace.

Ces moyens d'entraînement 5 peuvent être constitués par des bras articulés automatisés ou tous autres moyens équivalents connus de l'homme du métier.

La sonde 3 comprend une extrémité de terminaison 4 métallique, amovible et taillée en pointe pouvant être remplacée par une extrémité de terminaison 4 avec une pointe de dimension différente permettant d'effectuer des prélèvements précis sur la matière biologique 7.

La sonde 3 par l'action des moyens d'entraînement 5 se déplace au-dessus d'une culture de matière biologique 7, constituée ici de colonies bactériennes cultivées sur un milieu de culture 8 gélosé en boîte de Pétri 6.

La composition de la matière biologique 7 comprend essentiellement de l'eau liquide.

La sonde 3 comprend également un moyen de refroidissement (non illustré) pour givrer son extrémité de terminaison 4. Ce moyen peut par exemple être constitué par de l'azote liquide acheminé jusqu'à l'extrémité de terminaison 4 par un conduit situé dans la sonde 3 ou par la dépression d'un gaz réfrigérant dans un volume en contact avec l'extrémité de terminaison 4.

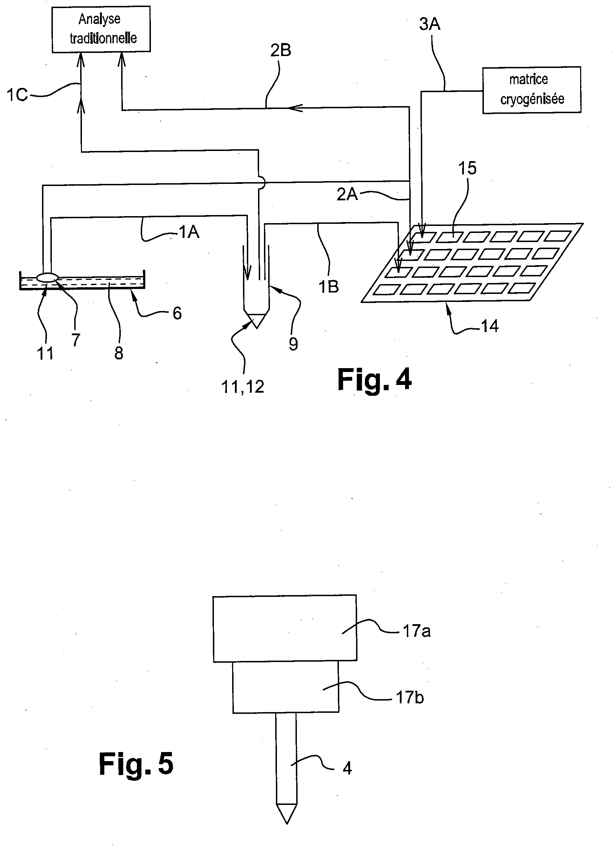

Il peut également être constitué par un ou plusieurs éléments Peltier en contact avec l'extrémité de terminaison 4. En particulier, la figure 5 illustre un mode de réalisation dans lequel les moyens de refroidissement comprennent deux étages 17a et 17b d'éléments Peltier sur lesquels est montée l'extrémité de terminaison 4.

Ces éléments présentent l'avantage d'être utilisés tant pour refroidir que pour chauffer l'extrémité de terminaison 4, en inversant leur tension électrique d'alimentation.

Afin d'éviter un échange thermique avec l'air ambiant, il peut être avantageux d'isoler la partie supérieure de l'extrémité de terminaison 4, qui n'entre pas en contact avec l'échantillon. Cette isolation permet d'optimiser l'action thermique de l'extrémité de terminaison 4.

Il est tout à fait envisageable de disposer de plus de deux éléments Peltier superposés. Les températures extrêmes obtenues seront d'autant plus importantes que le nombre d'éléments est important.

Ainsi, le refroidissement et le chauffage de l'extrémité de terminaison 4 seront plus rapides. Il peut alors être envisagé d'utiliser cette construction pour stériliser l'extrémité de terminaison en chauffant à une température importante. Ceci est particulièrement avantageux pour éviter des contaminations entre les différents échantillons de matière biologique, prélevés successivement.

Le cycle prélèvement/dépôt de matière biologique 7 peut ainsi être grandement accéléré et contrôlé.

La mise en oeuvre d'un procédé de prélèvement permet de coller à l'extrémité de terminaison 4 de la sonde 3 tout ou partie de l'échantillon 11 de matière biologique 7 à prélever ; dans ce cas présent, une colonie bactérienne cultivée sur le milieu de culture 8 en boîte de Pétri 6.

Dans une première mise en oeuvre du procédé de prélèvement, une pellicule de glace se dépose sur l'extrémité de terminaison 4 par le biais des moyens de refroidissement.

La formation de la pellicule de glace 13 est permise grâce à la présence de l'eau contenue dans l'air ambiant.

A cet effet, le dispositif 2 peut comporter directement une source cryogénique destinée à refroidir la sonde 3 et l'extrémité de terminaison 4.

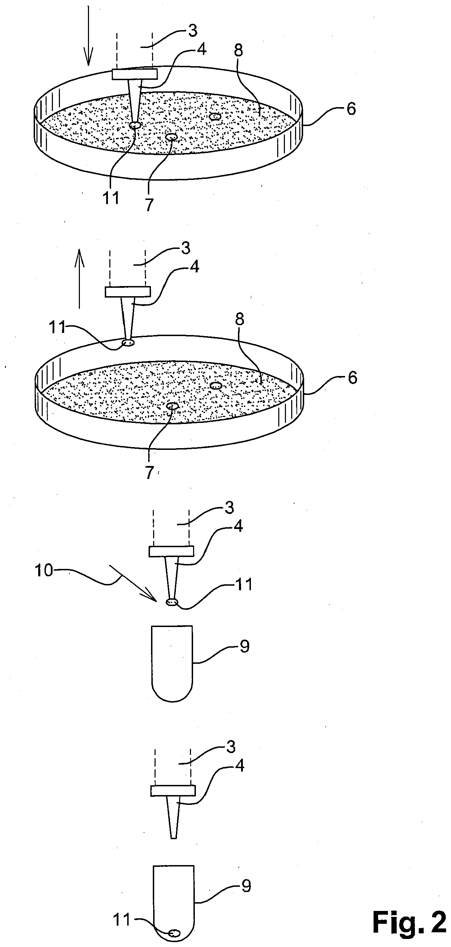

Comme illustré sur la figure 2, la sonde 3 avec son extrémité de terminaison 4 est amenée, par l'intermédiaire des moyens d'entraînement 5, au contact de la colonie 11 de bactéries 7, qui s'est développée sur la gélose 8 contenue dans la boîte de Pétri 6.

Ces moyens d'entraînements 5 par l'intermédiaire de l'extrémité de terminaison 4 appliquent une pression sur la colonie 11 à prélever. Cette pression associée à la basse température de la pellicule de glace 13 recouvrant l'extrémité de terminaison 4 permet de congeler l'eau contenue dans la colonie bactérienne 11.

Cette pression est mesurée par un capteur (non illustré) situé en dessous de la boîte de Pétri 6 et qui contrôle la descente des moyens d'entraînement 5, arrêtant cette descente au-delà d'un seuil de pression déterminé.

Alternativement, l'extrémité de terminaison 4 peut simplement venir au contact de la colonie 11, sans exercer de pression sur ladite colonie. Pour ce faire, il est utile de disposer d'un capteur de contact qui arrête la descente des moyens d'entrainement 5 dès que le contact est effectué. Un tel capteur est par exemple un capteur électrique binaire.

En congelant, l'eau contenue dans la colonie 11 forme un bloc solide avec la couche de glace 13 située à la surface de l'extrémité de terminaison 4 de la sonde 3. Tout ou partie de la colonie 11 à prélever est pris dans la glace et se retrouve alors collé sur l'extrémité de terminaison 4.

Le temps durant lequel la pression ou le contact est exercé peut être avantageusement contrôlé par le dispositif 2.

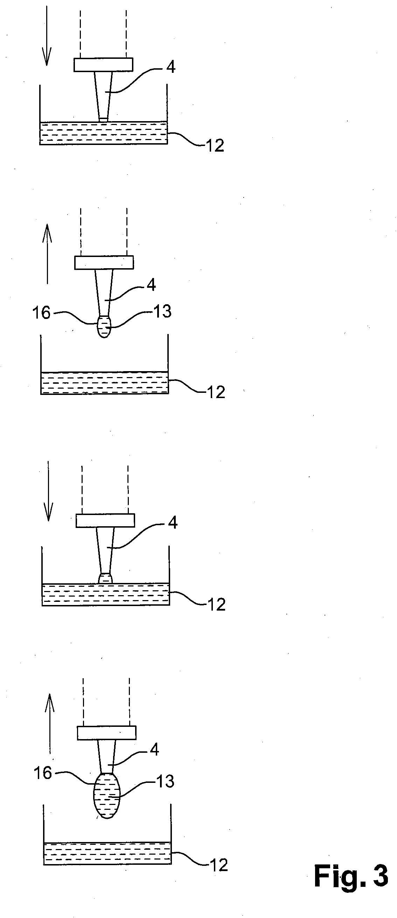

Dans une deuxième mise en oeuvre du procédé de prélèvement, illustrée à la figure 3, l'extrémité de terminaison 4 de la sonde 3 est mouillée dans une solution liquide 12, telle de l'eau liquide ou une matrice habituellement utilisée pour ioniser l'échantillon 11 de matière biologique 7 en vue de son analyse par un dispositif de mesure tel un spectromètre de masse.

La solution liquide 12 forme alors une couche de glace 13 généralement sous forme de goutte 16 à l'extrémité de l'extrémité de terminaison 4. La goutte 16 gèle sous l'action du moyen de refroidissement pour former une surface d'adhérence venant au contact, par le biais des moyens d'entraînement 5, de la colonie bactérienne 11 à prélever.

Dans une variante de cette deuxième mise en oeuvre du procédé de prélèvement également illustrée à la figure 3, l'extrémité de terminaison 4 de la sonde 3 est mouillée dans la solution liquide 12 plusieurs fois de suite afin d'agrandir la taille de la couche de glace 13 servant à l'adhérence des colonies 11 à prélever.

Ainsi, il est possible d'ajuster la taille de cette couche de glace 13 à la dimension de la colonie 11 à prélever.

Dans ces mises en oeuvre du procédé de prélèvement, il peut être avantageux de disposer d'une extrémité de terminaison 4 de sonde 3 présentant une forme apte à faciliter l'accrochage de la ou des goutte(s) de solution liquide 12, sur ladite extrémité. Cette forme peut être tronconique. L'extrémité peut par ailleurs présenter un rainure, par exemple horizontale, sur le pourtour de l'extrémité afin d'optimiser l'accrochage de la goutte de solution liquide 12.

Selon un mode particulier de réalisation, le conteneur contenant la solution liquide peut revêtir également une forme particulière. En premier lieu, il est avantageux de disposer d'un récipient de volume réduit, en adéquation avec le volume de la ou des gouttes(s) à former. En second lieu, l'intérieur du conteneur peut avantageusement revêtir une forme déterminée correspond à celle que doit revêtir la goutte. Ainsi, l'intérieur du récipient peut être de forme tronconique. Il est intéressant, par ailleurs, que la profondeur du conteneur soit importante dans la mesure où elle permet d'obtenir un goutte gelé relativement longue et donc ainsi, limiter les risques de contamination de l'extrémité de terminaison 4 par l'échantillon de matière biologique.

Selon un autre mode particulier de réalisation, le conteneur peut disposer de son propre moyen de refroidissement. Auquel cas, la solution liquide 12 contenue dans le conteneur est refroidie. En maintenant ladite solution liquide a une température proche de sa température de congélation, il est possible d'obtenir une formation plus rapide de la goutte gelée, lorsque l'extrémité de terminaison est trempée dans la solution liquide.

Dans une troisième mise en oeuvre du procédé de prélèvement (non représentée), une goutte de solution liquide 12 est déposée sur la colonie 11 à prélever, de sorte que cette dernière est totalement ou partiellement recouverte de liquide. L'extrémité de terminaison 4 de la sonde 3 est alors appliquée sur la goutte de solution liquide 12 de sorte que cette dernière se retrouve gelée emprisonnant et tout ou partie de la colonie 11 à prélever et gelant cette dernière à son tour.

Lors d'une étape ultérieure, les moyens d'entraînement 5 séparent tout ou partie de la colonie 11 de la couche de gélose 8 réalisant ainsi l'étape de prélèvement.

Les moyens d'entraînements 5 permettent d'amener la colonie 11 prélevée au-dessus d'un contenant 9, tel un tube à essai 9 ou d'une plaque d'analyses 14, telle une plaque d'analyses utilisée en spectrométrie de masse.

La mise en oeuvre d'un procédé de dépôt permet par la suite de séparer l'extrémité de terminaison 4 givrée de la sonde 3 et tout ou partie de la colonie 11 collée sur celle-ci.

Dans cette première mise en oeuvre de ce procédé de dépôt, la séparation entre la colonie 11 et l'extrémité de terminaison 4 est effectuée sans contact avec un quelconque support à l'air ambiant après interruption de l'action du moyen de refroidissement. En effet, une fois l'action du moyen de refroidissement interrompue, la glace constituée au niveau de l'extrémité de terminaison 4 de la sonde 3, fond libérant ainsi la colonie collée à elle. Il se forme alors une goutte, de volume plus ou moins important selon la quantité de glace formée initialement, dans laquelle les bactéries se retrouvent en suspension.

Dans une variante de cette première mise en oeuvre du procédé de dépôt selon l'invention, un moyen de chauffage 10 contribue à faire fondre plus rapidement la glace retenant la colonie 11 à l'encontre de l'extrémité de terminaison 4.

Ce moyen de chauffage 10 peut être constitué indifféremment par une flamme, un poste de soudage à air chaud, des rayons lumineux convergents, l'effet joule produit par le passage d'un courant dans un composant électrique résistif ou tout autre moyen équivalent produisant de la chaleur.

Ce moyen de chauffage 10 peut également être utilisé par la suite comme moyen de stérilisation pour stériliser l'extrémité de terminaison 4 de la sonde 3.

De la même façon que pour la première mise en oeuvre du procédé de dépôt décrite ci-dessus, la colonie 11 prélevée à partir de la deuxième mise en oeuvre du procédé de prélèvement décrite ci-dessus est également séparée de l'extrémité de terminaison 4, par l'intermédiaire d'un moyen de chauffage 10.

Dans cette mise en oeuvre du procédé de dépôt, la colonie 11 peut également être séparée de l'extrémité de terminaison 4 de la sonde 3 par l'application d'un choc mécanique sur la couche de glace 13.

Ce choc mécanique entraîne une rupture de la couche de glace 13. Celle-ci est alors recueillie en même temps que la colonie 11 prélevée dans un tube à essai 9. Un tel tube peut alors être exploité par exemple par un automate d'analyses tel que l'automate VITEK^{®} 2 ou une plaque d'analyses 14.

Selon une deuxième mise en oeuvre du procédé de dépôt, tout ou partie de la colonie 11 est déposée par le contact de l'ensemble ou partie de la colonie 11 sur la plaque d'analyses 14, ce contact provoquant la fonte progressive de la couche de glace 13 superficielle de l'échantillon par l'action sur la couche de glace 13 de la tension superficielle de la surface du contenant 9 ou de la plaque d'analyses 14.

Ce contact peut être séquentiel sous forme de spots distribués sur la plaque d'analyses 14 ou bien continus sous forme de traits.

Le dépôt sous forme de spots permet d'obtenir une pluralité de dépôts différents provenant du même échantillon de matière biologique (colonie 11) prélevé. Ces spots peuvent alors être exploités par exemple par un spectromètre de masse.

Le dépôt sous forme de traits continus permet d'obtenir un gradient de concentration en matière biologique 7 avec des concentrations plus fortes en début de dépôt qu'à la fin du dépôt. Il est ainsi possible de réaliser des mesures sur différentes zones du gradient de concentration, afin de localiser la zone au niveau de laquelle les résultats d'analyse sont les meilleurs, lors d'acquisitions de mesures par le spectromètre de masse.

La figure 4 illustre quelques moyens de mise en oeuvre des procédés de prélèvement et de dépôt.

Dans une application 1A correspondant au procédé de prélèvement, l'intégralité ou une partie de la colonie bactérienne 11 est directement congelée puis prélevée dans sa boîte de Pétri 6 par la sonde 3 pour être déposée ensuite dans un tube à essai 9 dans lequel les bactéries vont se retrouver en suspension après liquéfaction du glaçon formé par la sonde 3 autour de son extrémité de terminaison 4, en vue de subir une analyse traditionnelle.

Dans une application 1 B, une partie de la suspension bactérienne contenue dans le tube à essai 9 est prélevée pour être déposée sur la plaque d'analyse 14.

Dans une application 1C, une partie de la suspension bactérienne contenue dans le tube à essai 9 est prélevée pour être déposée sur un autre support en vue d'une seconde analyse traditionnelle, par exemple un test de sensibilité aux antibiotiques.

Dans une application 2A, la colonie bactérienne 11 ou une partie de cette colonie est directement congelée puis prélevée dans la boîte de Pétri 6.

Une partie de ce prélèvement est déposé directement sur la plaque d'analyses 14 d'un spectromètre de masse tandis qu'une autre partie, dans une application 2B, est déposée sur un autre support en vue d'une seconde analyse traditionnelle, par exemple un test de sensibilité aux antibiotiques.

Le prélèvement peut également être conservé sous forme congelée sur la sonde 3 afin de différer la seconde analyse. Ceci est facilité lorsque l'extrémité de terminaison 4 est amovible. En effet, la conservation de l'extrémité de terminaison 4 sous forme gelée peut par exemple être assurée par disposition de cette extrémité à l'intérieur d'un congélateur.

La plaque d'analyses 14 utilisée pour réaliser l'analyse en spectrométrie de masse de l'ensemble ou partie de la colonie 11 contient une matrice 15 utilisée pour ioniser le prélèvement.

Dans un moyen de mise en oeuvre particulier, la matrice 15 se présente sous forme d'un dépôt séché, après répartition sous forme de spots sur la plaque d'analyses 14.

La liquéfaction du dépôt gelé de l'ensemble ou partie de la colonie 11 au contact de la plaque d'analyses 14, permet la remise en suspension des éléments de la matrice 15 dans un mélange liquide et homogène comprenant également les bactéries.

Une autre solution illustrée par l'application 3A, consiste à venir prélever la colonie 11 dans la boîte de Pétri 6 après avoir mouillée l'extrémité de terminaison 4 de la sonde 3 directement dans la matrice 15 se présentant sous sa forme normale à l'état liquide, formant un glaçon de matrice 15 autour de l'extrémité de terminaison 4 de la sonde 3.

La liquéfaction du dépôt gelé au contact de la plaque d'analyses 14, composé de l'ensemble ou partie de la colonie 11 prélevée par la sonde 3 et de la matrice 15, va alors remettre en suspension les éléments de la matrice 15 dans un mélange liquide et homogène comprenant également les bactéries.

Dans ces deux derniers modes de réalisation, il est important de s'assurer préalablement que la concentration en éléments de matrice d'ionisation sera suffisante en regard du nombre de bactéries présentes dans le mélange.

En outre, des colonies 11 prélevées par ces procédés, utilisant une solution liquide non toxique tel que de l'eau, et réensemencées sur une boite de Pétri 6 repoussent, et ne sont donc pas tuées par ces procédés de prélèvement et de dépôt.

Des exemples d'applications industrielles sont proposés ci-dessous.

### EXEMPLES

### Exemple 1 :

Dans ce premier exemple, une pointe de glace est utilisée pour prélever une colonie de microorganisme inconnu, et pour la déposer sur une cible de spectromètre de masse afin d'identifier la colonie de microorganismes inconnus à l'aide d'un spectromètre de masse selon le protocole suivant :
- prélever la colonie sur gélose au sang (bioMérieux réf) avec une aiguille de glace,
- déposer avec la pointe de glace la colonie sur une cible de spectromètre de masse à 384 positions (Bruker). Le dépôt est obtenu par application brève de la pointe de glace sur la cible à température ambiante. Un léger film d'eau et de microorganismes est ainsi déposer à la surface de la cible.
- déposer 2 µl de matrice (acide alpha cyano dissout à 10mg/ml dans une solution d'acétone et d'eau 50/50) à la surface de l'échantillon.
- introduire la cible dans un spectromètre de masse de type MALDI-TOF (Ultraflex II, Bruker),
- régler le spectromètre de masse pour optimiser l'acquisition des masses entre 2000 et 20000 Da. L'homme de l'art a notamment l'habitude de régler les tensions et la puissance laser de l'instrument.
- calibrer le spectromètre de masse avec des protéines de référence (ProteinMixte, Bruker).
- analyser l'échantillon à l'aide de 20 séries de 50 tirs laser. Le spectre de masse obtenu pour chaque série est sommé pour obtenir le spectre de masse du microorganisme. Le nombre de tirs peut être ajusté par l'homme de l'art pour obtenir un spectre le plus informatif possible, c'est à dire avec le plus de pics de masses possibles et le mieux définis,
- déterminer les masses observées sur le spectre de masse du microorganisme à l'aide du logiciel Flex Analysis (Bruker),
- comparer les masses observées sur le spectre de masse du microorganisme avec les masses contenues dans une base de données. Plusieurs bases de données existent à cette fin : la demanderesse dispose de sa propre base, les bases des sociétés Bruker (Biotyper) ou Anagnostec (Saramis) peuvent également être utilisées.
- identifier le microorganisme présentant les masses les plus proche de celles observées sur le spectre de masse du microorganisme inconnu.

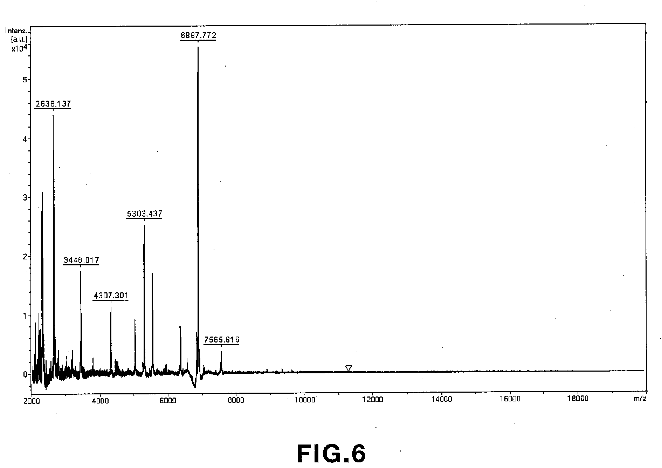

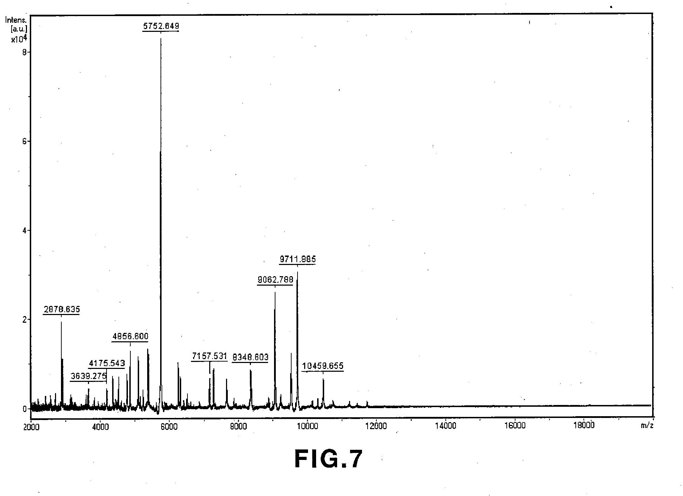

Pour cet exemple, ce protocole a été appliqué à deux colonies distinctes et a permis d'obtenir les spectres de masse visibles sur les figures 6 et 7.

Le spectre de masse de la figure 6 a permis d'identifier la colonie comme étant une colonie de *Staphylococcus aureus.*

Le spectre de masse de la figure 7 a permis d'identifier la colonie comme étant une colonie d'*Escherichia coli.*

Ce procédé est avantageux car il permet d'effectuer le prélèvement et le dépôt extrêmement rapidement avec un excellent taux de réussite. Dès le premier essai, toutes les colonies, quelle que soit leur nature, sont prélevées puis déposées avec succès par l'aiguille de glace.

De plus, la cible étant à température ambiante, l'aiguille de glace fond très légèrement lorsqu'elle touche la surface. Le léger filet d'eau qui en résulte entraine l'échantillon et permet d'obtenir un dépôt fin et homogène de microorganismes.

Ce dernier point est particulièrement avantageux car un dépôt épais entraine une suppression de signal en spectrométrie de masse. Ce phénomène, bien connu, est dû à un excès de sels et à une proportion de matrice inadaptée à la quantité d'échantillon. Un dépôt hétérogène est également préjudiciable, le signal devient hétérogène, ce qui rend difficile le réglage du spectromètre de masse.

### Exemple 2 :

Dans un deuxième exemple, le même protocole que pour l'exemple 1 est mis en oeuvre, à la différence près qu'à l'étape 2 le dépôt est obtenu en frottant la pointe de glace à la surface de la cible du spectromètre de masse.

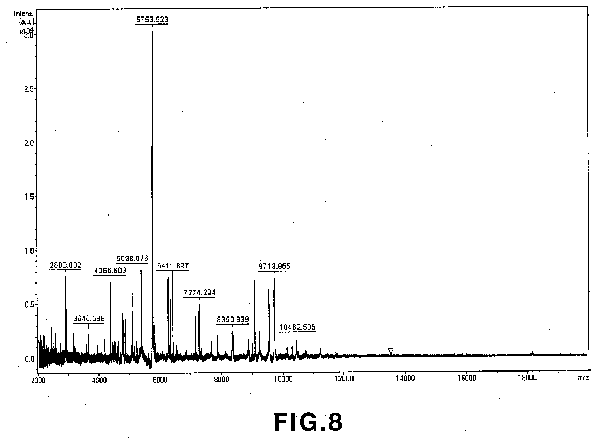

Le spectre figurant sur la figure 8 est obtenu. Ce spectre conduit à identifier *Escherichia coli.*

Ce protocole a les mêmes avantages que l'exemple 1. Il permet de surcroît un dépôt sur une plus grande surface, ce qui peut être utile pour réaliser plusieurs acquisitions successives du même échantillon, ou pour acquérir successivement plusieurs spectres de masse avec des paramètres différents.

### Exemple 3 :

Dans un troisième exemple, 25 souches d'espèces connues sont mises en culture en boite de Pétrie avec un milieu de culture COS (milieu Columbia avec sang de mouton, bioMérieux, référence 43041) ou SDA (millieu Sabouraud glucosée, bioMérieux, référence 43555) selon les indications figurant dans le tableau 1 ci-dessous :

**Tableau 1:**

| Milieu de culture | Référence bioMérieux du milieu de culture | Espèce de microorganisme | Référence bioMérieux du microorganisme |
|---|---|---|---|
| COS | 43041 | *Burkholderia multivorans* | 0301039 |
| COS | 43041 | *Proteus vulgaris* | 0509142 |
| COS | 43041 | *Pseudomonas putida* | 0509108 |
| COS | 43041 | *Streptococcus pseudopneumoniae* | 0507105 |
| COS | 43041 | *Bacillus licheniformis* | 0608016 |
| COS | 43041 | *Micrococcus luteus* | 0602045 |
| COS | 43041 | *Staphylococcus haemolyticus* | 0704061 |
| COS | 43041 | *Proteus mirabilis* | 0805068 |
| COS | 43041 | *Enterococcus raffinosus* | 0903030 |
| COS | 43041 | *Pseudomonas aeruginosa* | 1006028 |
| COS | 43041 | *Escherichia coli* | 1006021 |
| COS | 43041 | *Bacteroides fragilis* | 1009220 |
| COS | 43041 | *Shigella flexneri* | 7709005 |
| COS | 43041 | *Streptococcus pyogenes* | 7701086 |
| COS | 43041 | *Streptococcus constellatus ssp constella* | 7811150 |
| COS | 43041 | *Bacillus megaterium* | 8004066 |
| COS | 43041 | *Chryseobacterium indologenes* | 8105051 |
| COS | 43041 | *Vibrio parahaemolyticus* | 8305091 |
| COS | 43041 | *Citrobacter farmeri* | 8608073 |
| COS | 43041 | *Aeromonas hydrophila* | 606019) |
| COS | 43041 | *Salmonella ser. Gallinarum (pullovorum)* | 8703202 |
| COS | 43041 | *Corynebacterium jeikeium* | 9203007 |
| COS | 43041 | *Pseudomonas oryzihabitans* | 9510157 |
| COS | 43041 | *Enterococcus casseliflavus* | 9710016 |
| SDA | 43101 | *Geotrichum capitatum* | 9409060 |

- Après 16 heures de culture, pour chaque boite de Petri, une colonie est prélevée avec une aiguille de glace et déposée sur une cible de MALDI-TOF selon le procédé de l'invention. Cette opération est répétée pour obtenir deux dépôts indépendants pour chaque espèce étudiée. Les prélevements et les dépôts sont effectués selon le mode opératoire suivant.
- Une pointe métallique (extrémité de terminaison), de forme cylindrique à base tronconique, maintenue à -10°C par effet Peltier (pointe métallique en contact avec deux élements Peltier conformément à la figure 5). Elle est ensuite plongée pendant 15 secondes dans un bac d'eau maintenue à 9°C.
- La pointe métallique est sortie de l'eau. Une goutte d'eau adhère naturellement à la pointe métallique. Cette goutte d'eau congèle en 15 secondes par transfert de froid depuis la pointe métallique à -10°C. Une aiguille de glace est ainsi formée.
- L'aiguille de glace est posée sur une colonie de microorganismes et est aussitôt retirée. La colonie de microorganismes adhère instantanément à l'aiguille de glace et reste collée sur l'aiguille de glace. La colonie de microorganismes est ainsi prélevée par l'aiguille de glace. A l'inverse, la gélose du milieu de culture n'est pas arrachée.
- La colonie de microorganismes est déposée sur une cible jetable Fleximass DS à 48 positions de dépôt, de la société Shimadzu. Chaque position de dépôt est un cercle de 3mm de diametre. Le dépôt est effectué en frottant la pointe de glace sur la surface de l'une des 48 position de dépôt de la cible avec un mouvement en spiral. Ce mouvement est initié au centre de la position de dépôt et achevé après 4 tours de façon à couvrir l'ensemble de la position de dépôt d'un léger film d'eau et de microorganismes
- L'échantillon de microorganismes est séché à l'air libre quelques minutes.
- 1 µL de matrice (acide alpha cyano 4-hydroxycinnamique prêt à l'emploi en solution, (bioMérieux, référence 411071) est déposé à la surface de l'échantillon.
- L'échantillon de microorganismes et la matrtice sont séchés à l'air libre quelques minutes.
- La cible est introduite dans un spectromètre de masse de type MALDI-TOF (Axima Assurance, Shimadzu) avec un support de cible FLEXIMASS DS (Shimadzu).
- Le spectromètre de masse est réglé pour optimiser l'acquisition des masses entre 2000 et 20000 Da. L'homme de l'art a notamment l'habitude de régler les tensions et la puissance laser de l'instrument.
- Le spectromètre de masse est calibré avec un dépôt de *E. coli* (souche ATCC 8739).
- L'échantillon est analysé à l'aide de 100 séries de 5 tirs laser. Le spectre de masse obtenu pour chaque série est sommé pour obtenir le spectre de masse du microorganisme. Le nombre de tirs peut être ajusté par l'homme de l'art pour obtenir un spectre le plus informatif possible, c'est à dire avec le plus de pics de masse possibles et les mieux définis.
- les masses observées sur le spectre de masse du microorganisme sont déterminées à l'aide du logiciel LaunchPad version 2.8 (Shimadzu),
- Les masses observées sur le spectre de masse du microorganisme sont comparées avec les masses contenues dans la base de données Saramis (bioMérieux) à l'aide de l'interface Spectral ID version 1.1.0 (bioMérieux) et le microorganisme est identifié par comparaison avec les spectres de masse de microorganismes, présents dans la base de donnée.

Pour cet exemple, le protocole a permis d'identifier les dépôts selon les résultats figurant dans le tableau 2 ci-dessous :

**Tableau 2 :**

| | Dépôt n°1 | | Dépôt n°2 | |
|---|---|---|---|---|
| Espèce de microorganisme attendue | Espèce identifiée avec le protocole de l'exemple 3 | Probabilité d'identification (%) | Espèce identifiée avec le protocole de l'exemple 3 | Probabilité d'identification (%) |
| *Burkholderia multivorans* | *Burkhol.multivorans* | 100 | *Burkhol.multivorans* | 100 |
| *Proteus vulgaris* | *Proteus vulgaris* | 100 | *Proteus vulgaris* | 99,99 |
| *Pseudomonas putida* | *Ps.putida* | 99,99 | *Ps.putida* | 99,99 |
| *Streptococcus pseudopneumoniae* | *Pas d'identification* | - | *Str.pseudopneumoniae* | 65,67 |
| *Bacillus licheniformis* | *B.licheniformis* | 100 | *B.licheniformis* | 99,99 |
| *Micrococcus luteus* | *Mic.luteus*/*lylae* | 99,99 | *Mic.luteus*/*lylae* | 100 |
| *Staphylococcus haemolyticus* | *Staph.haemolyticus* | 99,99 | *Staph.haemolyticus* | 99,99 |
| *Proteus mirabilis* | *Proteus mirabilis* | 100 | *Proteus mirabilis* | 100 |
| *Enterococcus raffinosus* | *Entero.raffinosus* | 99,99 | *Entero.raffinosus* | 100 |
| *Pseudomonas aeruginosa* | *Ps.aeruginosa* | 99,99 | *Ps.aeruginosa* | 99,99 |
| *Escherichia coli* | *Esc. coli* | 99,99 | *Esch.coli* | 99,99 |
| *Bacteroides fragilis* | *Bac.fragilis* | 99,99 | *Bac.fragilis* | 99,99 |
| *Shigella flexneri* | *Esch.coli* | 99,99 | *Esch.coli* | 99,99 |
| *Streptococcus pyogenes* | *Str.pyogenes* | 100 | *Str.pyogenes* | 99,99 |
| *Streptococcus constellatus ssp constella* | *Str.constellatus* | 99,9 | *Pas d'identification* | - |
| *Bacillus megaterium* | *B.megaterium* | 99,99 | *B.megaterium* | 99,99 |
| *Chryseobacterium indologenes* | *Chryse.indologenes* | 99,99 | *Chryse.indologenes* | 99,99 |
| *Vibrio parahaemolyticus* | *V.parahaemolyticus* | 100 | *V.parahaemolyticus* | 99,99 |
| *Citrobacter farmeri* | *Citro.farmeri* | 99,99 | *Citro.farmeri* | 99,99 |
| *Aeromonas hydrophila* | *Aer.salm.salmonicida ou Aer.hydro.*/*caviae* | 99,99 ou 99,73 | *Aer.salm.salmonicida ou Aer.hydro.*/*caviae* | 99,99 ou 98,16 |
| *Salmonella ser. Gallinarum (pullovorum)* | *Salmonella group* | 78,25 | *Salmonella group* | 78,15 |
| *Corynebacterium jeikeium* | *Coryn.jeikeium* | 100 | *Coryn.jeikeium* | 100 |
| *Pseudomonas oryzihabitans* | *Ps.oryzihabitans* | 99,99 | *Ps.oryzihabitans* | 99,99 |
| *Enterococcus casseliflavus* | *Entero.casseliflavus* | 100 | *Pas d'identification* | - |
| *Geotrichum capitatum* | *G.capitatum* | 100 | *G.capitatum* | 100 |

L'espèce identifiée correspond exactement à l'espèce attendue en tenant compte des commentaires ci-dessous :
- Spectral ID fournit le nom abrégé de l'espèce. L'homme de l'art a l'habitude de ces abbrieviations. A titre d'exemple, il est pour lui évident que *G.capitatum* signifie *Geotrichum capitatum,* qu'*Entero.casseliflavus* signifie *Enterococcus casseliflavus*...
- *Micrococcus luteus* est identifié *Mic.luteus*/*lylae*, c'est à dire comme pouvant être *Micrococcus luteus* ou *Micrococcus lylae.* Ces deux espèces ne sont pas différentiables avec une analyse MALDI-TOF et la base de donnée Saramis.
- *Shigella flexneri* est identifiée comme étant *Esch.coli*, c'est à dire *Escherichia coli.* Comme précédemment, *Shigella flexneri* et *Escherichia coli* sont deux espèces trop proches pour pouvoir être distinguées par MALDI-TOF. Spectral ID et Saramis classent ces deux espèces comme étant *Escherichia coli.*
- *Aeromonas hydrophila* est identifiée comme *Aer.salm.salmonicida ou Aer.hydro.*/*caviae.* Les 3 espèces sont très proches et difficilement différentiables par MALDI-TOF.
- *Salmonella ser. Gallinarum (pullovorum)* est identifée comme *Salmonella* group. L'analyse MALDI-TOF est insuffisament résolutive pour distinguer sans équivoque le serovar *Gallinarum (pullovorum*) des autres serovars. Le logiciel Spectral ID et Saramis indiquent donc uniquement une identification au niveau du genre *Salmonella.*

Ainsi le protocole a permis d'identifier avec succès l'ensemble des espèces analysées. 72 dépôts sur 75 ont été identifiés, ce qui représente 97.3% d'identification correcte. Ce taux d'identification est très bon, voire même meilleur que ce que l'homme de l'art a l'habitude d'obtenir avec un prélévement et un dépôt manuel suivi d'une analyse MALDI-TOF.

Ce procédé est avantageux car il permet d'effectuer le prélèvement et le dépôt extrêmement rapidement avec un excellent taux de réussite et pour tout type de microorganisme (bactérie ou levure (*G.capitatum*)). Notamment, ce procédé permet de prélever et de déposer avec la même efficacité des microorganismes présentant des formes et des consistances très différentes (*Proteus, Bacillus*, coliformes...).

Bien que l'invention ait été décrite en liaison avec des exemples particuliers de mise en oeuvre et de réalisation, il est bien évident qu'elle n'y est nullement limitée et qu'elle comprend tous les équivalents techniques des étapes et moyens décrits ainsi que leurs combinaisons.

## Revendications

1. Procédé de prélèvement de tout ou partie d'un échantillon (11) de matière biologique (7), brut, enrichi ou cultivé au contact d'un milieu de culture (8), éventuellement un milieu de culture gélosé, utilisant une sonde (3) pourvu d'une extrémité de terminaison (4), ledit procédé de prélèvement comprenant les étapes de :
- refroidissement de l'extrémité de terminaison (4) de la sonde (3),
- collage de tout ou partie de l'échantillon (11) de matière biologique (7) à prélever par contact de l'extrémité de terminaison (4) sur l'échantillon (11) de matière biologique (7) ou par l'application d'une pression exercée par l'extrémité de terminaison (4) sur l'échantillon (11) de matière biologique (7), et
- prélèvement de tout ou partie de l'échantillon (11) de matière biologique (7) de façon à séparer l'échantillon (11) de matière biologique (7) de son support, tel qu'un milieu de culture (8).

2. Procédé de prélèvement selon la revendication 1, dans lequel préalablement à l'étape de collage, il est réalisé une étape de mouillage de l'extrémité de terminaison (4) de la sonde (3) dans une solution liquide (12), afin de former une couche de glace (13) sur l'extrémité de terminaison (4).

3. Procédé de prélèvement selon l'une des revendication 1 à 2, dans lequel préalablement à l'étape de collage, l'échantillon (11) de matière biologique (7) à prélever est recouvert d'une solution liquide (12), afin de former une couche de glace (13) autour de l'échantillon (11) lors de l'étape de collage de tout ou partie dudit échantillon (11) avec l'extrémité de terminaison (4) de la sonde (3).

4. Procédé de prélèvement selon l'une des revendication 2 à 3, dans lequel la solution liquide (12) est prise dans le groupe comprenant l'eau, une solution saline, un tampon, un milieu de culture liquide ou une matrice habituellement utilisée pour ioniser l'échantillon (11) de matière biologique (7) en vue de son analyse par un dispositif de mesure tel un spectromètre de masse.

5. Procédé de prélèvement selon l'une des revendications 2 à 4, dans lequel l'étape de mouillage de l'extrémité de terminaison (4) de la sonde (3) ou de l'échantillon (11) de matière biologique (7) à prélever dans une solution liquide (12) est répétée successivement au moins deux fois afin de former une superposition de couches de glace (13).

6. Procédé de prélèvement selon l'une des revendications 1 à 5, dans lequel l'extrémité de terminaison (4) est amovible.

7. Procédé de prélèvement selon la revendication 6, dans lequel l'extrémité de terminaison (4) possède des propriétés ferromagnétiques.

8. Procédé de prélèvement selon la revendication 7, dans lequel l'étape de prélèvement de tout ou partie de l'échantillon (11) de matière biologique (7) consiste à appliquer un champ magnétique, par exemple à l'aide d'un électro-aimant, au voisinage de l'extrémité de terminaison (4) ferromagnétique de façon à attirer l'extrémité de terminaison (4) et ainsi la récupérer.

9. Procédé de dépôt dans un contenant (9) ou sur une plaque d'analyses (14) de tout ou partie d'un échantillon (11) de matière biologique (7) collé sur une extrémité de terminaison (4) givrée d'une sonde, ledit procédé de dépôt comportant une étape de séparation de l'extrémité de terminaison (4) de la sonde (3) et de tout ou partie de l'échantillon (11) de matière biologique (7), collé sur ladite extrémité de terminaison (4).

10. Procédé de dépôt selon la revendication 9, dans lequel l'étape de séparation est réalisée sans contact de l'échantillon (11) de matière biologique (7) collé sur l'extrémité de terminaison (4) givrée avec le contenant (9) ou la plaque d'analyses (14).

11. Procédé de dépôt selon la revendication 9, dans lequel l'étape de séparation de l'extrémité de terminaison (4) et de tout ou partie de l'échantillon (11) de matière biologique (7) collé sur ladite extrémité de terminaison (4) est réalisée par l'application d'un choc mécanique sur l'extrémité de terminaison (4).

12. Procédé de dépôt selon la revendication 10, dans lequel l'étape de séparation de l'extrémité de terminaison (4) et de l'ensemble ou partie de l'échantillon (11) de matière biologique (7) collé sur l'extrémité de terminaison (4) givrée est réalisée par un moyen de chauffage (10) ou à l'air ambiant.

13. Procédé de dépôt selon la revendication 9, dans lequel l'étape de séparation est réalisée par le contact séquentiel de l'échantillon (11) de matière biologique (7) avec le contenant (9) ou la plaque d'analyses (14), ce contact provoquant la fonte d'une couche superficielle de glace (13) et le dépôt de matière biologique (7).

14. Procédé de dépôt selon la revendication 13, dans lequel tout ou partie de l'échantillon (11) de matière biologique (7) est distribué sur une plaque d'analyses (14) de manière à réaliser plusieurs dépôts distincts de parties d'un même échantillon (11) de matière biologique (7), collé sur l'extrémité de terminaison (4).

15. Procédé de dépôt selon la revendication 9, dans lequel le dépôt est effectué par le contact continu par exemple sous forme de traits de tout ou partie de l'échantillon (11) de matière biologique (7) collé sur l'extrémité de terminaison (4) avec le contenant (9) ou la plaque d'analyses (14).

16. Dispositif de prélèvement et de dépôt (2) de tout ou partie d'un échantillon (11) de matière biologique (7) brut, enrichi ou cultivé au contact d'un milieu de culture (8) et destiné à être déposé dans un contenant (9) ou une plaque d'analyses (14), **caractérisé en ce qu'**il comprend :
- une sonde (3) pourvue d'une extrémité de terminaison (4),
- un moyen de refroidissement configuré à givrer l'extrémité de terminaison (4),
- des moyens d'entraînement (5) configurés
• à exercer une pression de la sonde (3) sur l'échantillon (11) de manière à congeler tout ou partie de l'eau contenue dans l'échantillon (11) afin de le coller à l'extrémité de terminaison (4),
• à séparer tout ou partie de l'échantillon (11) de son support, tel qu'un milieu de culture (8),
• à rapprocher tout ou partie de l'échantillon (11) du contenant (9) ou de la plaque d'analyses (14).

17. Dispositif (2) selon la revendication 16, comprenant un moyen de chauffage (10) destiné à décoller l'échantillon (11) de l'extrémité de terminaison (4).

18. Dispositif (2) selon la revendication 17, dans lequel le moyen de chauffage (10) est destiné également à stérilisant l'extrémité de terminaison (4).

19. Dispositif (2) selon l'une des revendications 16 à 18, dans lequel le moyen de refroidissement et le moyen de chauffage sont constitués par au moins un élément Peltier.

20. Dispositif (2) selon l'une des revendications 16 à 19, dans lequel l'extrémité de terminaison (4) est métallique ou minérale.

21. Dispositif (2) selon l'une des revendications 16 à 20, dans lequel l'extrémité de terminaison (4) est au moins partiellement recouverte d'un revêtement ou d'un traitement hydrophobe.

22. Dispositif (2) selon l'une des revendications 16 à 21, dans lequel l'extrémité de terminaison (4) est amovible.

23. Dispositif (2) selon l'une des revendications 16 à 22, dans lequel l'extrémité de terminaison présente une forme optimisant le collage de tout ou partie de l'échantillon (11) de matière biologique (7) à prélever ou de la solution liquide (12).

24. Dispositif (2) selon l'une des revendications 16 à 23, dans lequel l'extrémité de terminaison (4) comprend une extrémité en pointe.

25. Dispositif (2) selon l'une des revendications 16 à 24, comprenant au moins un capteur contrôlant la pression exercée par les moyens d'entraînement (5) par l'intermédiaire de la sonde (3) en contact avec l'échantillon (11) de matière biologique (7) à prélever cultivé sur le milieu de culture (8), et ce afin de stopper cette pression.

26. Dispositif (2) selon la revendication 25, dans lequel le capteur est un capteur de pression ou de force.

27. Dispositif (2) selon l'une des revendications 16 à 26, comprenant un capteur détectant le contact entre la terminaison (4) et la matière biologique (7) afin d'éviter toute pression.

28. Dispositif (2) selon l'une des revendications 26 à 27, dans lequel le capteur est un capteur électrique binaire.

29. Kit comprenant un dispositif selon l'une des revendications 16 à 28 comportant une pluralité d'extrémités de terminaison (4) interchangeables.

30. Appareil (1) d'analyse médicale comprenant un dispositif selon l'une des revendications 16 à 28 ou un kit selon la revendication 29.

## Patentansprüche

1. Verfahren zur Entnahme einer gesamten oder eines Teils einer Probe (11) von biologischem Material (7), das roh, angereichert oder in Kontakt mit einem Kulturmedium (8), gegebenenfalls einem Agar-Kulturmedium, gezüchtet worden ist, unter Verwendung einer Sonde (3), die mit einem Abschlussende (4) ausgestattet ist, wobei das Entnahmeverfahren die folgenden Schritte umfasst:
- Kühlen des Abschlussendes (4) der Sonde (3),
- Anheften der gesamten oder eines Teils der zu entnehmenden Probe (11) von biologischem Material (7) durch Kontakt des Abschlussendes (4) auf der Probe (11) von biologischem Material (7) oder durch Anlegen eines Drucks, der von dem Abschlussende (4) auf die Probe (11) von biologischem Material (7) ausgeübt wird, und
- Entnehmen der gesamten oder eines Teils der Probe (11) von biologischem Material (7) derart, dass die Probe (11) von biologischem Material (7) von ihrem Träger, wie einem Kulturmedium (8), abgetrennt wird.

2. Entnahmeverfahren nach Anspruch 1, wobei vor dem Anheftungsschritt ein Schritt der Benetzung des Abschlussendes (4) der Sonde (3) in einer flüssigen Lösung (12) durchgeführt wird, um eine Eisschicht (13) auf dem Abschlussende (4) zu bilden.

3. Entnahmeverfahren nach einem der Ansprüche 1 bis 2, wobei vor dem Anheftungsschritt die zu entnehmende Probe (11) von biologischem Material (7) mit einer flüssigen Lösung (12) bedeckt wird, um eine Eisschicht (13) um die Probe (11) während des Anheftungsschritts der gesamten oder eines Teils der Probe (11) an dem Abschlussende (4) der Sonde (3) zu bilden.

4. Entnahmeverfahren nach einem der Ansprüche 2 bis 3, wobei die flüssige Lösung (12) aus der Wasser, eine Salzlösung, einen Puffer, ein flüssiges Kulturmedium oder eine Matrix, die üblicherweise zum lonisieren der Probe (11) von biologischem Material (7) für die Analyse mit einem Messgerät, wie einem Massenspektrometer, verwendet wird, umfassenden Gruppe stammt.

5. Entnahmeverfahren nach einem der Ansprüche 2 bis 4, wobei der Schritt der Benetzung des Abschlussendes (4) der Sonde (3) oder der zu entnehmenden Probe (11) von biologischem Material (7) in einer flüssigen Lösung (12) mindestens zweimal nacheinander wiederholt wird, um übereinander liegende Eisschichten (13) zu bilden.

6. Entnahmeverfahren nach einem der Ansprüche 1 bis 5, wobei das Abschlussende (4) abnehmbar ist.

7. Entnahmeverfahren nach Anspruch 6, wobei das Abschlussende (4) ferromagnetische Eigenschaften besitzt.

8. Entnahmeverfahren nach Anspruch 7, wobei der Schritt der Entnahme der gesamten oder eines Teils der Probe (11) von biologischem Material (7) aus dem Anlegen eines Magnetfelds, zum Beispiel mithilfe eines Elektromagneten, in der Nachbarschaft zu dem ferromagnetischen Abschlussende (4) besteht, so dass das Abschlussende (4) angezogen und damit zurückgewonnen wird.

9. Verfahren zum Absetzen einer gesamten oder eines Teils einer Probe (11) von biologischem Material (7), die auf einem vereisten Abschlussende (4) einer Sonde angeheftet ist, in einen Behälter (9) oder auf eine Analysenplatte (14), wobei das Absetzverfahren einen Schritt der Trennung des Abschlussendes (4) der Sonde (3) und der gesamten oder eines Teils der Probe (11) von biologischem Material (7), das auf dem Abschlussende (4) angeheftet ist, beinhaltet.

10. Absetzverfahren nach Anspruch 9, wobei der Trennungsschritt ohne Kontakt der auf dem vereisten Abschlussende (4) angehefteten Probe (11) von biologischem Material (7) mit dem Behälter (9) oder der Analysenplatte (14) durchgeführt wird.

11. Absetzverfahren nach Anspruch 9, wobei der Schritt der Trennung des Abschlussendes (4) von der gesamten oder einem Teil der Probe (11) von biologischem Material (7), die auf dem Abschlussende (4) angeheftet ist, durch Anwenden eines mechanischen Stoßes auf das Abschlussende (4) durchgeführt wird.

12. Absetzverfahren nach Anspruch 10, wobei der Schritt der Trennung des Abschlussendes (4) von der gesamten oder einem Teil der Probe (11) von biologischem Material (7), die auf dem vereisten Abschlussende (4) angeheftet ist, durch eine Heizeinrichtung (10) oder an der Umgebungsluft durchgeführt wird.

13. Absetzverfahren nach Anspruch 9, wobei der Trennungsschritt durch aufeinanderfolgenden Kontakt der Probe (11) von biologischem Material (7) mit dem Behälter (9) oder der Analysenplatte (14) durchgeführt wird, wobei dieser Kontakt das Schmelzen einer oberflächlichen Eisschicht (13) und das Absetzen des biologischen Materials (7) bewirkt.

14. Absetzverfahren nach Anspruch 13, wobei die gesamte oder ein Teil der Probe (11) von biologischem Material (7) auf einer Analysenplatte (14) verteilt wird, so dass mehrere getrennte Absetzungen von Teilen derselben Probe (11) von biologischem Material (7), die auf dem Abschlussende (4) angeheftet ist, erhalten werden.

15. Absetzverfahren nach Anspruch 9, wobei das Absetzen durch kontinuierlichen Kontakt, zum Beispiel in Form von Linien, der gesamten oder eines Teils der Probe (11) von biologischem Material (7), die auf dem Abschlussende (4) angeheftet ist, mit dem Behälter (9) oder der Analysenplatte (14) durchgeführt wird.

16. Vorrichtung zum Entnahme und zum Absetzen (2) einer gesamten oder eines Teils einer Probe (11) von biologischem Material (7), das roh, angereichert oder in Kontakt mit einem Kulturmedium (8) gezüchtet worden und dazu bestimmt ist, in einem Behälter (9) oder einer Analysenplatte (14) abgesetzt zu werden, **dadurch gekennzeichnet, dass** sie Folgendes umfasst:
- eine mit einem Abschlussende (4) ausgestattete Sonde (3),
- eine Kühleinrichtung, die für die Vereisung des Abschlussendes (4) eingerichtet ist,
- Antriebsmittel (5), die dafür eingerichtet sind:
• einen Druck der Sonde (3) auf die Probe (11) auszuüben, so dass das gesamte oder ein Teil des in der Probe (11) enthaltenen Wassers gefroren wird, um sie an das Abschlussende (4) anzuheften,
• die gesamte oder einen Teil der Probe (11) von ihrem Träger, wie einem Kulturmedium (8), zu trennen,
• die gesamte oder einen Teil der Probe (11) mit dem Behälter (9) oder der Analysenplatte (14) zusammenzubringen.

17. Vorrichtung (2) nach Anspruch 16, die eine Heizeinrichtung (10) zum Ablösen der Probe (11) von dem Abschlussende (4) umfasst.

18. Vorrichtung (2) nach Anspruch 17, wobei die Heizeinrichtung (10) auch zum Sterilisieren des Abschlussendes (4) bestimmt ist.

19. Vorrichtung (2) nach einem der Ansprüche 16 bis 18, wobei die Kühleinrichtung und die Heizeinrichtung aus mindestens einem Peltier-Element bestehen.

20. Vorrichtung (2) nach einem der Ansprüche 16 bis 19, wobei das Abschlussende (4) metallisch oder anorganisch ist.

21. Vorrichtung (2) nach einem der Ansprüche 16 bis 20, wobei das Abschlussende (4) zumindest zum Teil mit einer hydrophoben Beschichtung oder Bearbeitung bedeckt ist.

22. Vorrichtung (2) nach einem der Ansprüche 16 bis 21, wobei das Abschlussende (4) abnehmbar ist.

23. Vorrichtung (2) nach einem der Ansprüche 16 bis 22, wobei das Abschlussende eine Form besitzt, die die Anheftung der gesamten oder eines Teils der zu entnehmenden Probe (11) von biologischem Material (7) oder der flüssigen Lösung (12) optimiert.

24. Vorrichtung (2) nach einem der Ansprüche 16 bis 23, wobei das Abschlussende (4) ein spitzes Ende aufweist.

25. Vorrichtung (2) nach einem der Ansprüche 16 bis 24, die mindestens einen Sensor umfasst, der den von den Antriebsmitteln (5) über die Sonde (3) in Kontakt mit der zu entnehmenden Probe (11) von biologischem Material (7), das auf dem Kulturmedium (8) gezüchtet wird, ausgeübten Druck kontrolliert und zwar, um diesen Druck zu beenden.

26. Vorrichtung (2) nach Anspruch 25, wobei es sich bei dem Sensor um einen Druck- oder Kraftsensor handelt.

27. Vorrichtung (2) nach einem der Ansprüche 16 bis 26, die einen Sensor umfasst, der den Kontakt zwischen dem Abschluss (4) und dem biologischen Material (7) erfasst, um jeglichen Druck zu vermeiden.

28. Vorrichtung (2) nach einem der Ansprüche 26 bis 27, wobei es sich bei dem Sensor um einen binären elektrischen Sensor handelt.

29. Kit, das eine Vorrichtung nach einem der Ansprüche 16 bis 28 umfasst, die eine Mehrzahl gegeneinander austauschbare Abschlussenden (4) aufweist.

30. Medizinisches Analysegerät (1), das eine Vorrichtung nach einem der Ansprüche 16 bis 28 oder ein Kit nach Anspruch 29 umfasst.

## Claims

1. A method of taking all or part of a sample (11) of biological matter (7), which is crude, enriched or cultured through contact with a culture medium (8), possibly a gelose culture medium, using a probe (3) equipped with a terminal end (4), said sampling method comprising the steps of:
- cooling of the terminal end (4) of the probe (3),
- sticking all or part of the sample (11) of biological matter (7) to be taken through contact of the terminal end (4) onto the sample (11) of biological matter (7), or by applying a pressure exerted by the terminal end (4) onto the sample (11) of biological matter (7), and
- sampling all or part of the sample (11) of biological matter (7) so as to separate the sample (11) of biological matter (7) from its support, such as a culture medium (8).

2. The taking method according to claim 1, wherein prior to the sticking step, a step of wetting the terminal end (4) of the probe (3) in a liquid solution (12) is carried out, in order to form a layer of ice (13) on the terminal end (4).

3. The taking method according to one of claims 1 to 2, wherein prior to the sticking step, the sample (11) of biological matter (7) to be taken is covered with a liquid solution (12), in order to form a layer of ice (13) around the sample (11) during the step of sticking all or part of said sample (11) to the terminal end (4) of the probe (3).

4. The taking method according to one of claims 2 to 3, wherein the liquid solution (12) is taken from the group comprising water, a saline solution, a buffer, a liquid culture medium or a matrix commonly used for ionising the sample (11) of biological matter (7) with a view to analysing it using a measuring device such as a mass spectrometer.

5. The taking method according to one of claims 2 to 4, wherein the step of wetting the terminal end (4) of the probe (3) or the sample (11) of biological matter (7) to be taken in a liquid solution (12) is repeated successively at least twice in order to form overlaying layers of ice (13).

6. The taking method according to one of claims 1 to 5, wherein the terminal end (4) is removable.

7. The taking method according to claim 6, wherein the terminal end (4) possesses ferromagnetic properties.

8. The taking method according to claim 7, wherein the step of sampling all or part of the sample (11) of biological matter (7) consists in applying a magnetic field, for example using an electromagnet, adjacent to the ferromagnetic terminal end (4) so as to attract the terminal end (4) and thus recover it.

9. A method of depositing into a container (9) or onto an analysis plate (14) all or part of a sample (11) of biological matter (7) stuck onto a frosted terminal end (4) of a probe, said depositing method including a step of separating the terminal end (4) of the probe (3) from all or part of the sample (11) of biological matter (7) stuck onto said terminal end (4).

10. The depositing method according to claim 9, wherein the separation step is carried out without the sample (11) of biological matter (7) stuck on the frosted terminal end (4) coming into contact with the container (9) or the analysis plate (14).

11. The depositing method according to claim 9, wherein the step of separating the terminal end (4) from all or part of the sample (11) of biological matter (7) stuck on said terminal end (4) is carried out by applying a mechanical shock onto the terminal end (4).

12. The depositing method according to claim 10, wherein the step of separating the terminal end (4) from all or part of the sample (11) of biological matter (7) stuck on the frosted terminal end (4) is carried out by a heating means (10) or in ambient air.

13. The depositing method according to claim 9, wherein the separation step is achieved by sequential contact of the sample (11) of biological matter (7) with the container (9) or with the analysis plate (14), which thereby melts a superficial layer of ice (13) and deposits biological matter (7).

14. The depositing method according to claim 13, wherein all or part of the sample (11) of biological matter (7) is distributed on an analysis plate (14) so as to accomplish several distinct deposits of parts of the same sample (11) of biological matter (7), which is stuck on the terminal end (4).

15. The depositing method according to claim 9, wherein the depositing is effected through continuous contact, for example in the form of lines, of all or part of the sample (11) of biological matter (7) stuck on the terminal end (4) with the container (9) or the analysis plate (14).

16. A device for taking and depositing (2) all or part of a sample (11) of biological matter (7), which is crude, enriched or cultured through contact with a culture medium (8), and configured to be deposited into a container (9) or onto an analysis plate (14), **characterised in that** it comprises:
- a probe (3) equipped with a terminal end (4),
- a cooling means configured for frosting the terminal end (4),
- driving means (5) configured:
• for exerting a pressure from the probe (3) onto the sample (11) so as to freeze all or part of the water contained in the sample (11) in order to stick it to the terminal end (4),
• for separating all or part of the sample (11) from its support, such as a culture medium (8),
- to bring all or part of the sample (11) to the container (9) or the analysis plate (14).

17. The device (2) according to claim 16, comprising a heating means (10) configured for detaching the sample (11) from the terminal end (4).

18. The device (2) according to claim 17, wherein the heating means (10) is also configured for sterilising the terminal end (4).

19. The device (2) according to one of claims 16 to 18, wherein the cooling means and the heating means are composed of at least one Peltier element.

20. The device (2) according to one of claims 16 to 19, wherein the terminal end (4) is metallic or mineral.

21. The device (2) according to one of claims 16 to 20, wherein the terminal end (4) is at least partially covered with a hydrophobic coating or treatment.

22. The device (2) according to one of claims 16 to 21, wherein the terminal end (4) is removable.

23. The device (2) according to one of claims 16 to 22, wherein the terminal end has a shape which optimises the sticking of all or part of the sample (11) of biological matter (7) to be taken or of the liquid solution (12).

24. The device (2) according to one of claims 16 to 23, wherein the terminal end (4) comprises a pointed end.

25. The device (2) according to one of claims 16 to 24, comprising at least one sensor which controls the pressure exerted by the driving means (5) via the probe (3) in contact with the sample (11) of biological matter (7) to be taken which is cultured on the culture medium (8), and does this in order to halt this pressure.

26. The device (2) according to claim 25, wherein the sensor is a pressure or force sensor.

27. The device (2) according to one of claims 16 to 26, comprising a sensor which detects the contact between the terminal (4) and the biological matter (7) in order to avoid any pressure.

28. The device (2) according to one of claims 26 to 27, wherein the sensor is a binary electric sensor.

29. A kit comprising a device according to one of claims 16 to 28 including a plurality of interchangeable terminal ends (4).

30. A medical analysis apparatus (1) containing a device according to one of claims 16 to 28, or a kit according to claim 29.
